(19) Europäisches Patentamt
European Patent Office
Office européen des brevets

(11) EP 1 847 297 A2

(12) **DEMANDE DE BREVET EUROPEEN**

(43) Date de publication:
**24.10.2007 Bulletin 2007/43**

(21) Numéro de dépôt: **07105999.2**

(22) Date de dépôt: **12.04.2007**

(51) Int Cl.:
***A61Q 5/06*** *(2006.01)* ***A61K 8/40*** *(2006.01)*
***A61K 8/81*** *(2006.01)*

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **13.04.2006 FR 0603284**

(71) Demandeur: **L'Oréal**
**75008 Paris (FR)**

(72) Inventeurs:
- **BRUN, Gaëlle**
  **75015, PARIS (FR)**
- **GOURLAOUEN, Luc**
  **92600, ASNIERES (FR)**
- **BONNAMY, Arnaud**
  **78000, VERSAILLES (FR)**

(74) Mandataire: **Dossmann, Gérard**
**Bureau Casalonga & Josse**
**Bayerstrasse 71/73**
**80335 München (DE)**

(54) **Composition cosmétique comprenant au moins un monomère cyanoacrylate, au moins une particule organique thermostable non colorée et un solvant organique liquide.**

(57) La présente invention concerne une composition cosmétique pour le traitement des fibres kératiniques, en particulier des fibres kératiniques humaines telles que les cheveux, comprenant, au moins un solvant organique liquide, au moins un monomère cyanoacrylate polymérisable et au moins une particule organique thermostable non colorée ayant une taille primaire moyenne en nombre inférieure à 30 µm, à l'exception des particules de polytétrafluoroéthylène.

EP 1 847 297 A2

## Description

La présente invention concerne une composition cosmétique pour le traitement des fibres kératiniques, en particulier des fibres kératiniques humaines telles que les cheveux, comprenant, au moins un monomère cyanoacrylate polymérisable, au moins une particule organique thermostable non colorée et au moins un solvant organique liquide, une utilisation de cette composition pour le traitement des cheveux ainsi qu'un procédé de traitement la mettant en oeuvre.

Les cheveux sont généralement abîmés et fragilisés par l'action des agents atmosphériques extérieurs tels que la lumière et les intempéries, et par des traitements mécaniques ou chimiques tels que le brossage, le peignage, les décolorations, les permanentes et/ou les teintures. I1 en résulte que les cheveux sont souvent difficiles à discipliner, en particulier ils sont difficiles à démêler ou à coiffer, et les chevelures, même abondantes, conservent difficilement une coiffure de bon aspect en raison du fait que les cheveux manquent de vigueur, de volume et de nervosité.

Ainsi, pour remédier à cela, il est maintenant usuel d'utiliser des produits de coiffage qui permettent de conditionner les cheveux en leur apportant notamment du corps, de la masse ou du volume.

Ces produits de coiffage sont généralement des compositions cosmétiques capillaires comprenant un ou plusieurs polymères qui présentent une forte affinité pour les cheveux et qui ont le plus souvent pour fonction de former un film à leur surface en vue de modifier leurs propriétés superficielles, notamment pour les conditionner.

Un inconvénient lié à l'utilisation de ces compositions capillaires réside dans le fait que les effets cosmétiques conférés par de telles compositions ont tendance à disparaître, notamment dès le premier shampooing.

Afin de remédier à cet inconvénient, il est envisageable d'accroître la rémanence du dépôt de polymères en effectuant directement une polymérisation radicalaire de certains monomères au niveau des cheveux. Toutefois, les traitements ainsi obtenus entraînent une dégradation de la fibre et les cheveux ainsi traités sont généralement difficilement démêlables.

Par ailleurs, il est connu du document FR 2 833 489 d'utiliser des monomères électrophiles polymérisant par voie anionique directement à la surface des cheveux en présence d'un agent nucléophile tel que des ions hydroxyde ($OH^-$) contenus dans l'eau à pH neutre. Ainsi, une fois déposés sur la chevelure, ces monomères forment un polymère conduisant à un gainage satisfaisant.

Cependant, le gainage obtenu à partir de ces compositions ne présente pas une résistance satisfaisante par rapport aux diverses agressions extérieures que peuvent subir les cheveux.

I1 existe donc un réel besoin de trouver des compositions cosmétiques, notamment pour le conditionnement des cheveux, qui soient rémanentes aux shampooings et aux agressions extérieures tout en conservant de bonnes propriétés cosmétiques, c'est-à-dire d'apporter du corps, de la masse ou du volume aux cheveux et ceci de manière durable.

Ainsi la présente invention a pour objet une composition cosmétique pour le traitement des fibres kératiniques, en particulier des fibres kératiniques humaines, telles que les cheveux qui comprend, au moins un solvant organique liquide, au moins un monomère cyanoacrylate polymérisable et au moins une particule organique thermostable non colorée ayant une taille primaire moyenne en nombre inférieure à 30 μm, à l'exception des particules de polytétrafluoroéthylène.

Ainsi, la composition de l'invention en associant des particules organiques thermostables non colorées ayant une taille primaire moyenne en nombre inférieure à 30 μm à un monomère cyanoacrylate, dans un solvant organique liquide permet d'apporter du corps, de la masse ou du volume aux fibres kératiniques notamment les cheveux de façon durable.

Ainsi, une telle composition permet d'obtenir un gainage renforcé qui présente une bonne résistance par rapport aux diverses agressions extérieures que peuvent subir les cheveux, notamment par rapport aux corps gras, tels que le sébum, et par rapport aux shampooings.

De plus, le revêtement obtenu se présente sous la forme d'un dépôt homogène, lisse et possède une excellente adhésion aux cheveux.

Par ailleurs, on a constaté également que les cheveux restaient parfaitement individualisés et pouvaient être coiffés sans problème.

L'invention a aussi pour objet un procédé de traitement cosmétique mettant en oeuvre la composition cosmétique selon l'invention.

Un autre objet de la présente invention consiste en une utilisation de la composition cosmétique pour le traitement des fibres kératiniques.

L'invention a encore pour objet un dispositif à plusieurs compartiments ou kits comprenant d'une part une composition comprenant au moins un monomère cyanoacrylate et d'autre part une composition comprenant au moins une particule organique thermostable non colorée tel que défini précédemment et au moins un solvant organique liquide.

D'autres objets, caractéristiques, aspects et avantages de l'invention apparaîtront encore plus clairement à la lecture de la description et des exemples qui suivent.

Le ou les monomères cyanoacrylate présents dans la composition de l'invention sont de préférence choisis

parmi les monomères de formule (I) :

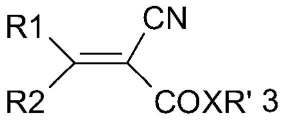

(I)

dans laquelle :

X désigne NH, S ou O,
R1 et R2 désignent chacun, indépendamment l'un de l'autre, un groupe peu ou non électro-attracteur (peu ou non inductif-attracteur) tel que :

- un atome d'hydrogène,
- un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR,
- OH, et les atomes d'halogène,
- un résidu polyorganosiloxane modifié ou non,
- un groupement polyoxyalkylène,

R désigne un groupe hydrocarboné saturé ou non, linéaire, ramifié ou cyclique, comportant de préférence de 1 à 20 atomes de carbone, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en $C_1$-$C_{10}$.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et / ou siloxy et/ou silanol et / ou amine et / ou imine et / ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que $-(CH_2)_n-(CF_2)_m-CF_3$ ou $-(CH_2)_n-(CF_2)_m-CHF_2$ avec n = 1 à 20 et m = 1 à 20.

Les substituants R1 et R2 peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoïques, quinoniques, méthiniques, cyanométhiniques et triarylméthane.

A titre d'exemples de groupement à fonction antioxydante, on peut notamment citer les groupements de type

butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et de type esters gras.

De préférence R1 et R2 représentent un atome d'hydrogène,

R'3 représentant un atome d'hydrogène ou un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en $C_1$-$C_{10}$..

De préférence R'3 est un groupe hydrocarboné saturé comportant de 1 à 10 atomes de carbone.

De préférence, X désigne O.

A titre de composés de formule (I), on peut citer les monomères :

a) appartenant à la famille des 2-cyanoacrylate de polyfluoroalkyle tels que :

l'ester 2,2,3,3-tétrafluoropropylique de l'acide 2-cyano-2-propénoïque de formule :

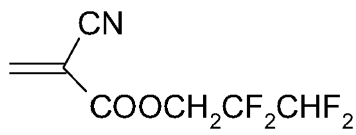

(II)

ou encore l'ester 2,2,2-trifluoroéthylique de l'acide 2-cyano-2-propénoïque de formule :

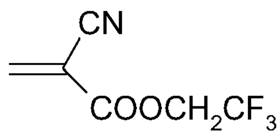

(III)

b) les 2-cyanoacrylate d'alkyle ou d'alcoxyalkyle

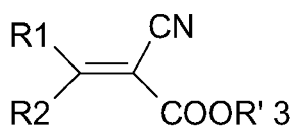

(IV)

dans laquelle R'3 représente un radical alkyle en C1-C10, alcényle en C2-C10, ou alcoxy(C1-C4) alkyle(C1-C10).

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle le 2-cyanoacrylate d'allyle, le 2-cyanoacrylate de méthoxypropyle et le cyanoacrylate d'iso-amyle.

Dans le cadre de l'invention, on préfère utiliser les monomères b). Selon un mode de réalisation préféré, le ou les monomères cyanoacrylates sont choisis parmi les cyanoacrylates d'alkyle en C6-C10.

Les monomères particulièrement préférés sont les cyanoacrylate d'octyle de formule V et leurs mélanges :

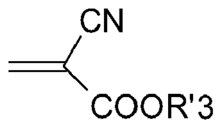

(V)

dans laquelle :

R'3 =$-(CH_2)_7-CH_3$,

$-CH(CH_3)-(CH_2)_5-CH_3$,

$-CH_2-CH(C_2H_5)-(CH_2)_3-CH_3$,

$-(CH_2)_5-CH(CH_3)-CH_3$,

$-(CH_2)_4-CH(C_2H_5)-CH_3$.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Le ou les monomères cyanoacrylates peut ou peuvent être présents dans une quantité allant de 0,1 à 80 % en poids, de préférence dans une quantité allant de 0,2 à 60 % en poids, et encore plus préférentiellement dans une quantité allant de 0,5 à 50 % en poids, par rapport au poids total de la composition cosmétique.

Dans le cadre de la présente invention, les monomères électrophiles cyanoacrylates de formule (I) sont des monomères capables de polymériser par voie anionique en présence d'un agent nucléophile.

Par polymérisation anionique, on entend le mécanisme défini dans l'ouvrage "Advanced Organic Chemistry", Third Edition de Jerry March, pages 151 à 161.

Les agents nucléophiles susceptibles d'initier la polymérisation anionique sont des systèmes connus en eux-mêmes, capables de générer un carbanion au contact d'un agent nucléophile, tels que les ions hydroxydes contenus dans l'eau à pH neutre. On entend par " carbanion ", les espèces chimiques définies dans "Advanced Organic Chemistry, Third Edition ", de Jerry March, page 141.

Les agents nucléophiles peuvent être appliqués indépendamment de la composition de l'invention. I1 peut aussi être ajouté à la composition de l'invention au moment de l'emploi.

L'agent nucléophile est un composé moléculaire, un oligomère, un dendrimère ou un polymère possédant des fonctions nucléophiles. De façon non limitative, on peut citer comme fonctions nucléophiles les fonctions : $R_2N^-$, $NH_2^-$, $Ph_3C^-$, $R_3C^-$, $PHNH^-$, pyridine, $ArS^-$, $R-C{\equiv}C^-$, $RS^-$, $SH^-$, $RO^-$, $R_2NH$, $ArO^-$, $N_3^-$, $OH^-$, $ArNH_2$, $NH_3$, $I^-$, $Br^-$, $Cl^-$, $RCOO^-$, $SCN^-$, ROH, RSH, $NCO^-$, $CN^-$, $NO_3^-$, $ClO_4^-$, $H_2O$, Ph représentant un groupe phényle ; Ar représentant un groupe aryle et R représentant un groupe alkyle en $C_1$-$C_{10}$.

Les monomères cyanoacrylates de formule (I) selon la présente invention peuvent être synthétisés selon les méthodes connues décrites dans la technique. En particulier, les monomères cyanoacrylates peuvent être synthétisés selon l'enseignement du US 3 527 224, US 3 591 767, US 3 667 472, US 3 995 641, US 4 035 334 et US 4 650 826.

La ou les particules utilisées dans la composition cosmétique selon la présente invention sont des particules organiques thermostables non colorées. De préférence, les particules sont polymériques et peuvent être issues de la polymérisation d'un ou de plusieurs monomères. Les polymères constituant ces particules organiques peuvent être réticulés ou non.

Au sens de la présente invention, on entend par « particules organiques thermostables », des particules organiques dont la taille ne varie pas de plus de 20 % lorsqu'elles sont soumises à un chauffage à 130 °C durant deux minutes.

Au sens de la présente invention, on entend par « particules organiques non colorées », des particules organiques qui lorsqu'elles sont déposées de manière jointive sur un support, c'est-à-dire que les particules sont au contact les unes des autres, présentent une différence de coloration avec l'étalon blanc de référence du colorimètre qui est inférieure ou égale à 10, et de préférence inférieure à 2.

La différence de coloration avec l'étalon blanc de référence du colorimètre est évaluée par l'écart de couleur

$$DE = \sqrt{(L_i*-L_0*)^2+(a_i*-a_0*)^2+(b_i*-b_0*)^2}$$

$L_i$*, $a_i$*, $b_i$* et $L_0$*, $a_0$*, $b_0$* étant respectivement les coordonnées colorimétriques dans le système La*b* des particules organiques et de l'étalon blanc de référence du colorimètre.

Avantageusement, la ou les particules organiques présentent une taille primaire moyenne en nombre comprise entre 0,1 et 30 μm, de préférence comprise entre 0,2 et 20 μm, et encore plus préférentiellement comprise entre 0,5 et 15 μm.

Au sens de la présente invention, on entend par « taille primaire de particule », la dimension maximale qu'il est possible de mesurer entre deux points diamétralement opposés d'une particule individuelle.

La taille des particules organiques peut être déterminée par microscopie électronique à transmission ou à partir de la mesure de la surface spécifique par la méthode BET ou à partir d'une granulométrie laser.

La ou les particules organiques utilisées dans la composition cosmétique peuvent présenter différentes formes, par exemple une forme de sphères, de paillettes, d'aiguilles ou de plaquettes et de préférence elles sont sensiblement sphériques.

La ou les particules organiques peuvent être pleines, creuses ou poreuses.

Lorsque les particules organiques sont creuses, elles comportent au moins une enveloppe continue (ou une couche superficielle) et au moins une cavité. L'enveloppe des particules est de préférence flexible pour se prêter à une déformation mécanique. Elle comprend généralement au moins un polymère, homo- ou copolymère, formé à partir de monomères à insaturation éthyléniques.

Les monomères utilisés peuvent être en particulier des esters d'acide méthacrylique ou acrylique, tels que l'acrylate et méthacrylate de méthyle, le chlorure de vinylidène, l'acrylonitrile, le styrène et ses dérivés.

Au sens de la présente invention, on entend par « particules poreuses », des particules ayant une structure comportant des pores en nombre et taille variables. La porosité associée à la taille des particules peut être caractérisée quantitativement à partir de la mesure de la surface spécifique par la méthode BET.

De préférence, les particules organiques poreuses présentent une surface spécifique supérieure ou égale à 1 $m^2$/g, de préférence supérieure ou égale à 2 $m^2$/g, et encore plus préférentiellement supérieure ou égale à 4 $m^2$/g.

La surface spécifique est déterminée selon la méthode BET (BRUNAUER-EMMET-TELLER) décrite dans « The journal of the Americain Chemical Society », vol.60, page 309 février 1938 et correspond à la norme internationale ISO 5794/1 (annexe D). La surface spécifique déterminée par la méthode BET correspond à la surface spécifique totale, micropores compris, des particules organiques considérées.

La ou les particules organiques utilisées dans la composition cosmétique selon l'invention peuvent être choisies parmi les poudres de polyamide, les poudres de polymères acryliques, notamment de polyméthacrylate de méthyle, les poudres de copolymères acryliques, notamment de polyméthacrylate de méthyle/diméthacrylate d'éthylène glycol, de polyméthacrylate d'allyle/diméthacrylate d'éthylène glycol, de copolymère diméthacrylate d'éthylène glycol/méthacrylate de lauryle, de polyacrylate/alkylacrylate, les poudres de polystyrène, les poudres de polyéthylène, notamment de polyéthylène/acide acrylique, les microbilles de résine de silicone.

A titre représentatif et non limitatif, on peut particulièrement citer en tant que particules organiques selon l'invention :

- les poudres de polyamides (Nylon ®), par exemples celles commercialisées sous les dénominations « ORGASOL ® 4000 » et « ORGASOL ® 2002 UD NAT COS 204 » par la société ATOCHEM,
- les poudres de polymères acryliques, notamment de polyméthacrylate de méthyle, comme par exemples celles commercialisées sous la dénomination « COVABEAD ® LH85 » « COVABEAD ® PMMA »par la société WACKHERR ou celles commercialisées sous la dénomination « MICROPEARL ® MHB » commercialisée par la société MATSUMOTO,
- les poudres de copolymères acryliques, notamment de polyméthacrylate de méthyle/diméthacrylate d'éthylène glycol, comme celles commercialisées sous la dénomination de « DOW CORNING 5640 MICROSPONGE ® SKIN OIL ADSORBER » par la société DOW CORNING, ou celles commercialisés sous la dénomination « GANZPEARL ® GMP-0820 » par la société GANZ CHEMICAL, de polyméthacrylate d'allyle/diméthacrylate d'éthylène glycol, comme celles commercialisées sous la dénomination « POLYPORE ® L200 » ou « POLYPORE ® E200 » commercialisés par la société AMCOL, de copolymère diméthacrylate d'éthylène glycol / méthacrylate de lauryle, comme celles commercialisées par « POLYTRAP ® 6603 » par la société DOW CORNING, de polyacrylate/éthylhexylacrylate comme celles commercialisées sous la dénomination « TECHPOLYMER ® ACX 806C » par la société SEKISUI,
- les poudres de polystyrène / dinvinylbenzène comme celles commercialisées sous la dénomination « TECHPOLYMER ® SBX8 » par la société SEKISUI,

- les poudres de polyéthylène, notamment de polyéthylène/acide acrylique commercialisées sous la dénomination « FLOBEADS ® » par la société SUMITOMO,
- les microbilles de résine de silicone, comme celles commercialisées sous les dénominations « TOSPEARL ® » par la société TOSHIBA SILICONE, en particulier les « TOSPEARL ® 240A » et « TOSPEARL ® 120A ».

De préférence, les particules organiques utilisées dans la composition conforme à l'invention sont choisies parmi les poudres de polyamide et les poudres de polyméthylméthacrylate.
La ou les particules organiques peuvent être éventuellement traitées en surface par un agent de traitement hydrophobe.
Ainsi les particules organiques peuvent être rendues hydrophobes par enrobage ou greffage chimique par des produits tels que :

- les silicones, comme les méthicones ou les diméthicones,
- les acides aminés, les acides aminés N-acylés ou leurs sels,
- les savons métalliques comme le dimyristate d'aluminium, le sel d'aluminium du glutamate de suif hydrogéné,
- les dérivés fluorés, comme par exemple les perfluoroalkyl phosphates, les perfluoroalkyl silanes, les polyoxydes d'hexafluoropropylène, les polyorganosiloxanes comprenant des groupes perfluroalkyl perfluoropolyéthers,
- la lécithine, le triisostéaroyl titanate d'isopropyle,
- les acides gras comme l'acide stéarique.

Le terme alkyle mentionné dans les composés cités ci-dessus peut désigner notamment un groupe alkyle, linéaire, ramifié ou cyclique comprenant de 1 à 30 atomes de carbone, notamment de 5 à 16 atomes de carbone.
Les acides aminés N-acylés peuvent comprendre un groupe acyle comprenant de 8 à 22 atomes de carbone, comme par exemple un groupe 2-éthyl hexanoyle, caproyle, lauroyle, myristoyle, palmitoyle, stéaroyle et cocoyle.
Les sels de ces composés peuvent être les sels d'aluminium, de magnésium, de calcium, de zirconium, de zinc de sodium ou de potassium.
L'acide aminé peut être par exemple la lysine, l'acide glutamique ou l'alanine.
A titre d'exemple, on peut citer les microsphères de poly méthacrylate de méthyle enrobées de tri-isostéaryl titanate d'isopropyle de taille 2-15 μm commercialisées par Kobo sous la référence BPA-5I5.
La ou les particules organiques thermostables non colorées utilisées dans la composition cosmétique conforme à l'invention peuvent être présentes en une quantité allant de 0,1 à 15 % en poids, et encore plus préférentiellement en une quantité allant de 0,2 à 10 % en poids, par rapport au poids total de la composition cosmétique.
Par solvant organique, on entend une substance organique capable de dissoudre une autre substance sans la modifier chimiquement.
Les solvants organiques liquides sont choisis parmi les composés liquides à la température de 25°C et sous 105 Pa (760mm de Hg) et sont différents des monomères cyanoacrylates de l'invention.
Le solvant organique est par exemple choisi parmi :

les alcools aromatiques tels que l'alcool benzylique ; les alcools gras liquides , notamment en $C_{10}$-$C_{30}$; les polyols modifiés ou non tels que le glycérol, le glycol, le propylène glycol, le dipropylène glycol, le butylène glycol, le butyle diglycol ; les silicones volatiles telles que la cylopentasiloxane, la cyclohexasiloxane, les polydiméthylsiloxanes modifiées ou non par des fonctions alkyle et/ou amine et/ou imine et/ou fluoroalkyl et/ou carboxylique et/ou betaïne et/ou ammonium quaternaire, les polydiméthylsiloxanes modifiées liquides, les huiles minérales, organiques ou végétales, les alcanes et plus particulièrement les alcanes de $C_5$ à $C_{10}$ ; les acides gras liquides, les esters gras liquides et plus particulièrement les benzoates ou les salicylates d'alcool gras liquides.

Le solvant organique est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ou encore des composés organiques tels que des alcanes en $C_5$-$C_{10}$, l'acétone, la méthyléthylcétone, les esters d'acides en $C_1$-$C_{20}$ liquides et d'alcools en $C_1$-$C_8$ tels que l'acétate de méthyle, l'acétate de butyle, l'acétate d'éthyle et le myristate d'isopropyle, le diméthoxyéthane, le diéthoxyéthane, les alcools gras liquides en $C_{10}$-$C_{30}$ tels que l'alcool oléique, les esters d'alcools gras en $C_{10}$-$C_{30}$ liquides tels que les benzoates d'alcool gras en $C_{10}$-$C_{30}$ et leurs mélanges ; l'huile de polybutène, l'isononanoate d'isononyle, le malate d'isostéaryle, le tétra-isostéarate de pentaérythrityle, le trimélate de tridécyle, le mélange cyclopentasiloxane (14,7% en poids)/polydiméthylsiloxane dihydroxylé en positions alpha et oméga (85,3% en poids), ou leurs mélanges.
Selon un mode de réalisation préféré, le solvant organique liquide est constitué par une silicone ou un mélange

de silicones tels que les polydiméthylsiloxanes liquides et les polydiméthylsiloxanes modifiées liquides, leur viscosité à 25°c est comprise entre 0.1cst et 1 000 000cst et plus préférentiellement entre 1 cst et 30 000cst.

On citera de préférence les huiles suivantes :

- le mélange de polydiméthylsiloxane alpha-omega-dihydroxylé/cyclopentadiméthylsiloxane (14,7/85,3) commercialisé par Dow Corning sous le nom de DC 1501 Fluid
- le mélange de polydiméthylsiloxane alpha-omega-dihydroxylé/ polydiméthylsiloxane commercialisé par Dow Corning sous le nom de DC 1503 Fluid
- le mélange de diméthicone /cyclopentadiméthylsiloxane commercialisé par Dow Corning sous le nom de DC 1411 Fluid ou celui commercialisé par Bayer sous le nom SF1214 ;
- la cyclopentadiméthylsiloxane commercialisée par Dow Corning sous le nom de DC245 Fluid ;

et les mélanges respectifs de ces huiles.

Le milieu des compositions cosmétiques de l'invention peut contenir, outre le ou les solvants organiques liquides, de l'eau.

De préférence, le milieu des composition est un milieu anhydre c'est-à-dire qu'il contient moins de 1% en poids d'eau par rapport au poids total de la composition.

Le ou les solvants organiques liquides de la composition représentent généralement de 0,01 à 99 %, de préférence de 50 à 99 % en poids par rapport au poids total de la composition.

La composition cosmétique selon l'invention peut comprendre en outre au moins un pigment.

L'utilisation d'un pigment dans la composition cosmétique conforme à l'invention permet d'obtenir des colorations visibles notamment sur des cheveux foncés puisque le pigment en surface masque la couleur naturelle de la fibre.

La composition conforme à l'invention présente ainsi l'avantage de conduire à des colorations qui présentent une bonne résistance aux diverses agressions que peuvent subir les cheveux, telles que les corps gras ou les shampooings.

De plus, la composition cosmétique selon l'invention permet de conduire à des colorations visibles et très chromatiques sur une fibre kératinique notamment foncée sans qu'il soit nécessaire d'éclaircir ou de décolorer les fibres kératiniques et, par conséquent, sans dégradation physique des fibres kératiniques.

Au sens de la présente invention, on entend par pigment toute entité organique et / ou minérale dont la solubilité dans l'eau est inférieure à 0,01 % à 20 °C, de préférence inférieure à 0,0001 % et présentant une absorption entre 350 et 700 nm, de préférence une absorption avec un maximum.

Les pigments organiques présentent une différence de coloration avec l'étalon blanc de référence du colorimètre supérieure à 10.

Les pigments utilisés dans la composition selon l'invention peuvent être notamment choisis parmi les pigments organiques et/ ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments conformes à l'invention peuvent par exemple être choisis parmi les pigments blancs ou colorés, les laques, les pigments à effets spéciaux tels que les nacres ou les paillettes, et leurs mélanges.

A titre d'exemples de pigments minéraux, on peut citer le dioxyde de titane, traité ou non traité en surface, les oxydes de zirconium ou de cérium, les oxydes de fer ou de chrome, le violet de manganèse, le bleu outremer, l'hydrate de chrome et le bleu ferrique. Par exemple, les pigments minéraux suivants peuvent être utilisés :

$Ta_2O_5$, $Ti_3O_5$, $Ti_2O_3$, TiO, $ZrO_2$ en mélange avec $TiO_2$, $ZrO_2$, $Nb_2O_5$, $CeO_2$, ZnS.

A titres d'exemples de pigments organiques, on peut citer les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références C1 42090, 69800, 69825, 73000, 74100, 74160, les pigments jaunes codifiés dans le Color Index sous les références C1 11680, 11710, 15985, 19140, 20040, 21100, 21108, 47000, 47005, les pigments verts codifiés dans le Color Index sous les références C1 61565, 61570, 74260, les pigments oranges codifiés dans le Color Index sous les références CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

On peut utiliser des pâtes pigmentaires de pigment organique telles que les produits vendus par la société HOECHST sous le nom :

- JAUNE COSMENYL IOG : Pigment YELLOW 3 (C1 11710) ;
- JAUNE COSMENYL G : Pigment YELLOW 1 (Cl 11680) ;
- ORANGE COSMENYL GR : Pigment ORANGE 43 (C1 71105) ;
- ROUGE COSMENYL R : Pigment RED 4 (C1 12085) ;
- CARMIN COSMENYL FB : Pigment RED 5 (Cl 12490) ;
- VIOLET COSMENYL RL : Pigment VIOLET 23 (C1 51319) ;
- BLEU COSMENYL A2R : Pigment BLUE 15.1 (C1 74160) ;
- VERT COSMENYL GG : Pigment GREEN 7 (Cl 74260) ;
- NOIR COSMENYL R : Pigment BLACK 7 (Cl 77266).

Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Par laque, on entend les colorants adsorbés sur des particules insolubles, l'ensemble ainsi obtenu restant insoluble lors de l'utilisation. Les substrats inorganiques sur lesquels sont adsorbés les colorants sont par exemple l'alumine, la silice, le borosilicate de calcium et de sodium ou le borosilicate de calcium et d'aluminium, et l'aluminium. Parmi les colorants organiques, on peut citer le carmin de cochenille.

A titre d'exemples de laques, on peut citer les produits connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 7 (CI 15 850:1), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments colorés qui procurent une teinte uniforme opaque, semi-transparente ou transparente classique.

A titre d'exemples de pigments à effets spéciaux, on peut citer les pigments nacrés blancs tels que le mica recouvert de titane, ou d'oxychlorure de bismuth, les pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre de pigments nacrés, on peut citer les nacres Cellini commercialisée par Engelhard (Mica-TiO2-laque), Prestige commercialisée par Eckart (Mica-TiO2), Prestige Bronze commercialisée par Eckart (Mica-Fe2O3), Colorona commercialisée par Merck (Mica-TiO2-Fe2O3).

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être fabriqués selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size séries of highly luminescent nanocristallites" Journal of phisical chemistry B, vol 101, 1997, pp 9463-9475. et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

La variété des pigments qui peuvent être utilisés dans la présente invention permet d'obtenir une riche palette de couleurs, ainsi que des effets optiques particuliers tels que des effets métalliques, interférentiels.

Selon un mode de réalisation particulier, les pigments sont des pigments colorés. On entend par pigment coloré des pigments autres que les pigments blancs.

La taille du pigment utile dans le cadre de la présente invention est généralement comprise entre 10 nm et 200 μm, de préférence entre 20 nm et 80 μm, et plus préférentiellement entre 30 nm et 50 μm.

Les pigments peuvent être enrobés par des composés organiques ou minéraux.

L'agent organique avec lequel sont traités les pigments peut être déposé sur les pigments par évaporation de solvant, réaction chimique entre les molécules de l'agent de surface ou création d'une liaison covalente entre l'agent de surface et les pigments ou les charges.

Le traitement en surface au sens de la présente invention est tel qu'un pigment traité en surface conserve ses propriétés intrinsèques de pigment avant traitement.

Le traitement en surface peut ainsi être réalisé par exemple par réaction chimique d'un agent de surface avec la surface des pigments et création d'une liaison covalente entre l'agent de surface et les pigments. Cette méthode est notamment décrite dans le brevet US 4 578 266.

De préférence, on utilisera un agent organique lié aux pigments de manière covalente.

L'agent pour le traitement de surface peut représenter de 0,1 à 50 % en poids du poids total des pigments ou des charges traités en surface, de préférence de 0,5 à 30 % en poids, et encore plus préférentiellement de 1 à 10 % en poids.

De préférence, les traitements en surface des pigments sont choisis parmi les traitements suivants :

- un traitement PEG-Silicone comme le traitement de surface AQ commercialisé par LCW ;
- un traitement Chitosane comme le traitement de surface CTS commercialisé par LCW ;
- un traitement Triéthoxycaprylylsilane comme le traitement de surface AS commercialisé par LCW ;
- un traitement Méthicone comme le traitement de surface SI commercialisé par LCW ;
- un traitement Diméthicone comme le traitement de surface Covasil 3.05 commercialisé par LCW ;
- un traitement Diméthicone / Triméthylsiloxysilicate comme le traitement de surface Covasil 4.05 commercialisé par LCW ;
- un traitement Lauroyl Lysine comme le traitement de surface LL commercialisé par LCW ;
- un traitement Lauroyl Lysine Diméthicone comme le traitement de surface LL / SI commercialisé par LCW ;
- un traitement Myristate de Magnésium comme le traitement de surface MM commercialisé par LCW ;
- un traitement Dimyristate d'Aluminium comme le traitement de surface MI commercialisé par Miyoshi ;
- un traitement Perfluoropolyméthylisopropyl éther comme le traitement de surface FHC commercialisé par LCW ;
- un traitement Isostéaryl Sébacate comme le traitement de surface HS commercialisé par Miyoshi ;
- un traitement Disodium Stéaroyl Glutamate comme le traitement de surface NAI commercialisé par Miyoshi ;
- un traitement Diméthicone / Disodium Stéaroyl Glutamate comme le traitement de surface SA / NAI commercialisé par Miyoshi ;
- un traitement Phosphate de Perfluoroalkyle comme le traitement de surface PF commercialisé par Daito ;
- un traitement Copolymère acrylate / Diméthicone et Phosphate de Perfluoalkyle comme le traitement de surface FSA commercialisé par Daito ;
- un traitement Polyméthylhydrogène siloxane / Phosphate de Perfluoroalkyle comme le traitement de surface FS01 commercialisé par Daito ;
- un traitement Lauryl Lysine / Aluminium Tristéarate comme le traitement de surface LL-StAl commercialisé par Daito ;
- un traitement Octyltriéthylsilane comme le traitement de surface OTS commercialisé par Daito ;
- un traitement Octyltriéthylsilane / Phosphate de Perfluoroalkyle comme le traitement de surface FOTS commercialisé par Daito ;
- un traitement Copolymère Acrylate / Diméthicone comme le traitement de surface ASC commercialisé par Daito ;
- un traitement Isopropyl Titanium Triisostéarate comme le traitement de surface ITT commercialisé par Daito ;
- un traitement Cellulose Microcrystalline et Carboxyméthyl Cellulose comme le traitement de surface AC commercialisé par Daito ;
- un traitement Cellulose comme le traitement de surface C2 commercialisé par Daito ;
- un traitement copolymère Acrylate comme le traitement de surface APD commercialisé par Daito ;
- un traitement Phosphate de Perfluoroalkyle / Isopropyl Titanium Triisostéarate comme le traitement de surface PF + ITT commercialisé par Daito.

Le ou les pigments sont chacun généralement présents dans la composition conforme à l'invention dans des quantités généralement comprises entre 0,05 et 50 % du poids total de la composition, de préférence de 0,1 à 35 % en poids.

Le milieu de la composition de l'invention peut aussi se présenter sous forme d'une émulsion et / ou être encapsulé, les monomères électrophiles étant maintenus dans un milieu anhydre jusqu'au moment de l'utilisation. Lorsque le milieu est une émulsion, cette émulsion est par exemple constituée par une phase dispersée ou continue qui peut être constituée par de l'eau, des alcools aliphatiques en C1-C4 ou leurs mélanges et une phase organique anhydre comprenant le monomère. Dans le cas des capsules ou microcapsules, la capsule peut contenir le monomère dans un milieu anhydre et être dispersées dans un milieu anhydre tel que défini précédemment, de l'eau, des alcools aliphatiques en C1-C4 ou leurs mélanges.

La composition de l'invention peut contenir des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, le trifluorure de bore, l'hydroquinone et ses dérivés tels que l'hydroquinone monéthyléther, la TBHQ, la benzoquinone et ses dérivés tels que la duroquinone, le catéchol et ses dérivés tels que le t-butyl catéchol et le méthoxycatéchol, l'anisole et ses dérivés tels que le méthoxyanisole ou l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, le 3-sulfonène et leurs mélanges. Les groupements alkyle désignent de préférence des groupement ayant 1 à 6 atomes de carbone.

On peut aussi utiliser à titre d'inhibiteur les acides minéraux ou organiques.

Ainsi la composition cosmétique selon l'invention peut également comprendre au moins un acide minéral ou organique, ce dernier ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique, l'acide octanoïque, l'acide heptanoïque et l'acide hexanoïque.

De préférence, l'acide acétique est utilisé.

La concentration en inhibiteur dans la composition cosmétique de l'invention peut être comprise entre 10 ppm et 30% en poids et plus préférentiellement entre 10 ppm et 15% en poids par rapport au poids total de la composition.

Pour moduler la cinétique de polymérisation anionique, on peut également augmenter la nucléophilie de la fibre par transformation chimique de la matière kératinique.

A titre d'exemple, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants:

- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

La composition de l'invention peut aussi contenir un ou plusieurs polymères ne présentant pas de réactivité sur les monomères cyanoacrylates et qui est capable d'augmenter la viscosité de la composition. L'augmentation de la viscosité permet de réduire la vitesse de polymérisation des monomères de cyanoacrylate. Pour ce faire, on peut ajouter à la composition de l'invention et de façon non exhaustive le polyméthylméthacrylate (PMMA) ou encore les copolymères à base de cyanoacrylate tels qu'ils sont décrits dans le brevet US 6,224,622.

Les compositions conformes à l'invention peuvent également contenir au moins un agent utilisé habituellement en cosmétique, choisi, par exemple, parmi des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des polymères fixants ou non, des polyols, des protéines, des vitamines, des colorants directs ou d'oxydation, des agents nacrants, des propulseurs, et des épaississants minéraux ou organiques tels que le benzylidène-sorbitol et les N-acylaminoacides, les cires oxyéthylénées ou non, les paraffines, les amides gras en C10-C30 tel que le diéthanolamide laurique et leurs mélanges.

Les compositions peuvent se présenter sous différentes formes galéniques tels qu'une lotion, une mousse

aérosol, un après shampooing ou un shampooing, un gel, une cire. Les compositions peuvent être contenues dans un flacon pompe, un spray aérosol. Les compositions de l'invention après application sur la chevelure peuvent être rincées ou non.

Lorsque la composition est contenue dans un aérosol, elle peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non (butane, propane, isobutane) et leurs mélanges.

Selon le procédé de l'invention, la composition de l'invention est appliquée sur les fibres kératiniques, en particulier les fibres kératiniques humaines telles que les cheveux, en présence d'un agent nucléophile.

Selon un mode de réalisation particulier du procédé de l'invention, l'agent nucléophile susceptible d'initier la polymérisation du monomère cyanoacrylate peut être appliqué au préalable sur les fibres kératiniques. L'agent nucléophile peut être utilisé pur, en solution, sous forme d'une émulsion ou être encapsulé. I1 peut aussi être ajouté à la composition anhydre au moment de l'emploi juste avant l'application sur les fibres kératiniques.

De préférence, cet agent nucléophile est l'eau. Cette eau peut être apportée par exemple par humidification préalable des fibres kératiniques. Elle peut aussi être ajoutée directement dans la composition avant application.

Selon un mode de réalisation particulier, il est possible de moduler la cinétique de polymérisation en humidifiant préalablement la fibre à l'aide d'une solution aqueuse dont le pH a été ajusté à l'aide d'une base, d'un acide ou d'un mélange acide/base. L'acide et/ou la base peuvent être inorganique ou organique.

Selon une variante, le procédé de traitement comprend une étape d'application des particules organiques thermostables non colorées sur les fibres kératiniques humaines, telles que les cheveux, suivie d'une étape d'application sur lesdites fibres d'au moins un monomère cyanoacrylate et du solvant organique liquide.

Selon encore une autre variante, le procédé de traitement des fibres kératiniques peut être un procédé de coloration capillaire pouvant être mis en oeuvre en plusieurs étapes : une première étape qui consiste à appliquer une composition contenant le ou les pigments sur les fibres et une seconde étape qui consiste à appliquer la composition selon l'invention contenant entre autres le monomère cyanoacrylate, la particule organique thermostable non colorée et le solvant organique liquide, l'agent nucléophile étant présent dans la composition contenant le pigment ou dans une composition séparée.

Selon cette variante, la composition cosmétique contenant le ou les pigments est de préférence une dispersion aqueuse de pigments ce qui permet une humidification de la fibre et l'initiation de la polymérisation lorsque la composition comprenant le monomère cyanoacrylate, la particule organique et le solvant organique liquide est appliquée.

Selon le procédé de l'invention, un mode de réalisation préféré consiste à appliquer le solvant organique liquide, le monomère cyanoacrylate, la particule organique et éventuellement les pigments à partir d'une même composition.

Le procédé de l'invention peut comprendre des étapes additionnels intermédiaires ou finales telles que l'application d'un produit cosmétique, une étape de rinçage, une étape de séchage. Le séchage peut être effectué au casque, au sèche cheveux et/ou au fer à lisser. En particulier, l'application des compositions conformes à l'invention peut être suivie d'un rinçage.

I1 est aussi possible de réaliser des applications multiples de la composition de l'invention afin d'obtenir une superposition de couches pour atteindre des propriétés spécifiques du dépôt en termes de nature chimique, résistance mécanique, épaisseur, aspect, toucher.

La présente invention concerne également l'utilisation d'une composition cosmétique telle que décrite précédemment pour le traitement des cheveux.

Lorsque la composition cosmétique selon l'invention ne comprend pas de pigments, la composition cosmétique peut être utilisée pour le conditionnement des cheveux.

Ainsi lorsque la composition cosmétique selon l'invention comprend à la fois la particule organique et un pigment, ladite composition peut être utilisée pour colorer les cheveux et éventuellement les conditionner.

L'invention a encore pour objet un dispositif à plusieurs compartiments ou kit, comprenant un premier compartiment, comprenant une composition contenant au moins un monomère cyanoacrylate polymérisable tel que défini précédemment et éventuellement au moins un inhibiteur de polymérisation anionique et/ou radicalaire, et un second compartiment, comprenant une seconde composition contenant, le solvant organique liquide et au moins une particule organique thermostable non colorée tels que définis précédemment, l'une des deux ou les deux compositions peuvent contenir un ou plusieurs additifs cosmétiques tels que définis précédemment.

Les exemples qui suivant sont destinés à illustrer l'invention, sans présenter un caractère limitatif.

**EXEMPLES**

Exemple 1

On a préparé une composition de coiffage (A) selon l'invention à partir des composés suivants :

Composition A

| | |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 45g |
| cyclopentadiméthylsiloxane commercialisé par Dow Corning sous le nom de DC245 Fluid | 41.75g |
| Microsphères creuses de polyméthylméthacrylate (surface spécifique 50 $m^2/g$) commercialisées sous la dénomination Covabead LH85 par LCW | 3g |
| Méthylheptylcyanoacrylate de Chemence | 10g |
| Acide acétique | 0,25 g |

0,3g de la composition est appliqué sur une mèche de 1g de cheveux propre et humide. Après 15 minutes de pause, la mèche est séchée au sèche-cheveux pendant 2 minutes. On obtient une mèche dont les cheveux sont individualisés qui a un toucher agréable et rémanent.

Composition B

On a préparé une composition de coiffage (B) selon l'invention à partir des composés suivants :

| | |
|---|---|
| DC 1501 Fluid | 45g |
| DC 245 Fluid | 41,75g |
| Microsphères de polyamide 12 (surface spécifique 9.5 $m^2/g$) commercialisées sous la dénomination Orgasol 2002 UD NAT COS 204 par Arkema | 3g |
| Méthylheptylcyanoacrylate de Chemence | 10g |
| Acide acétique | 0.25g |

0,3g de la composition est appliqué sur une mèche de 1g de cheveux propre et humide. Après 15 minutes de pause, la mèche est séchée au sèche-cheveux pendant 2 minutes. On obtient une mèche dont les cheveux sont individualisés qui a un toucher agréable et rémanent.

Composition C

On a préparé une composition de coiffage (C) selon l'invention à partir des composés suivants :

| | |
|---|---|
| DC 1501 Fluid | 45g |
| DC 245 Fluid | 41,75g |
| Microsphères du copolymère de polyméthyl méthacrylate et d'éthylèneglycol diméthacrylate commercialisées sous la dénomination Techpolymer MB30X-5 par Sekisui | 3g |
| Méthylheptylcyanoacrylate de Chemence | 10g |
| Acide acétique | 0.25g |

0,3g de la composition est appliqué sur une mèche de 1g de cheveux propre et humide. Après 15 minutes de pause, la mèche est séchée au sèche-cheveux pendant 2 minutes. On obtient une mèche dont les cheveux sont individualisés qui a un toucher agréable et rémanent.

Composition D

On a préparé une composition de coiffage (D) selon l'invention à partir des composés suivants :

| | |
|---|---|
| DC 1501 Fluid | 45g |
| DC 245 Fluid | 41.75g |
| Microsphères de polystyrène/divinylbenzene commercialisées sous la dénomination Techpolymer SBX-8 par Sekisui | 3g |
| Méthylheptylcyanoacrylate de Chemence | 10g |
| Acide acétique | 0.25g |

0,3g de la composition est appliqué sur une mèche de 1g de cheveux propre et humide. Après 15 minutes de pause, la mèche est séchée au sèche-cheveux pendant 2 minutes. On obtient une mèche dont les cheveux sont individualisés qui a un toucher agréable et rémanent.

Exemple 2

On a préparé une composition de coloration (E) selon l'invention à partir des composés suivants :

Composition E

| | |
|---|---|
| DC 1501 Fluid | 40g |
| DC 245 Fluid | 36,75g |
| Nacre mica enrobé d'oxyde de fer brun commercialisée sous la dénomination Prestige Bronze par Eckart | 10g |
| Microsphères creuses de polyméthylméthacrylate (surface spécifique 50 $m^2/g$) commercialisées sous la dénomination Covabead LH85 par LCW | 3g |
| Méthylheptylcyanoacrylate de Chemence | 10g |
| Acide acétique | 0,25g |

0,5g de la composition est appliqué sur une mèche de 1g de cheveux propre et humide. Après 15 minutes de pause, la mèche est séchée au sèche-cheveux pendant 2 minutes. On obtient une mèche colorée dont les cheveux sont individualisés qui a un toucher agréable et rémanent.

On a préparé une composition de coloration (F) selon l'invention à partir des composés suivants :

Composition F

| | |
|---|---|
| DC 1501 Fluid | 40g |
| DC 245 Fluid | 36.75g |
| Microsphères de polyamide 12 (surface spécifique 9.5 $m^2/g$) commercialisées sous la dénomination Orgasol 2002 UD NAT COS 204 commercialisées par Arkema | 3g |
| Nacre mica enrobé d'oxyde de fer brun commercialisée sous la dénomination Prestige Bronze par Eckart | 10g |
| Méthylheptylcyanoacrylate de Chemence | 10g |
| Acide acétique | 0.25g |

0,5g de la composition est appliqué sur une mèche de 1g de cheveux propre et humide. Après 15 minutes de pause, la mèche est séchée au sèche-cheveux pendant 2 minutes. On obtient une mèche colorée dont les cheveux sont individualisés qui a un toucher agréable et rémanent.

Composition G

On a préparé une composition de coloration (G) selon l'invention à partir des composés suivants :

| | |
|---|---|
| DC 1501 Fluid | 40g |
| DC 245 Fluid | 36.75g |
| Microsphères du copolymère de polyméthyl méthacrylate et d'éthylèneglycol diméthacrylate commercialisées sous la dénomination Techpolymer MB30X-5 par Sekisui | 3g |
| Nacre mica enrobé d'oxyde de fer brun commercialisée sous la dénomination Prestige Bronze par Eckart | 10g |
| Méthylheptylcyanoacrylate de Chemence | 10g |
| Acide acétique | 0.25g |

0,5g de la composition est appliqué sur une mèche de 1g de cheveux propre et humide. Après 15 minutes de pause, la mèche est séchée au sèche-cheveux pendant 2 minutes. On obtient une mèche colorée dont les cheveux sont individualisés qui a un toucher agréable et rémanent.

<u>Composition H</u>

On a préparé une composition de coloration (G) selon l'invention à partir des composés suivants :

| | |
|---|---|
| DC 1501 Fluid | 40g |
| DC 245 Fluid | 36.75g |
| Microsphères de polystyrène/divinylbenzene commercialisées sous la dénomination Techpolymer SBX-8 par Sekisui | 3g |
| Nacre mica enrobé d'oxyde de fer brun commercialisée sous la dénomination Prestige Bronze par Eckart | 10g |
| Méthylheptylcyanoacrylate de Chemence | 10g |
| Acide acétique | 0.25g |

0,5g de la composition est appliqué sur une mèche de 1g de cheveux propre et humide. Après 15 minutes de pause, la mèche est séchée au sèche-cheveux pendant 2 minutes. On obtient une mèche colorée dont les cheveux sont individualisés qui a un toucher agréable et rémanent.

**Revendications**

1. Composition cosmétique pour le traitement des fibres kératiniques, en particulier des fibres kératiniques humaines telles que les cheveux, **caractérisée par le fait qu'**elle comprend, au moins un solvant organique liquide, au moins un monomère cyanoacrylate polymérisable et au moins une particule organique thermostable non colorée ayant une taille primaire moyenne en nombre inférieure à 30 μm, à l'exception des particules de polytétrafluoroéthylène.

2. Composition cosmétique selon la revendication 1,
**caractérisée par le fait que** le monomère cyanoacrylate est choisi parmi les monomères de formule (I) :

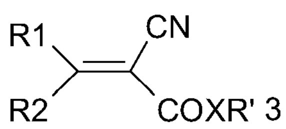

(I)

dans laquelle :

X désigne NH, S ou O,
R1 et R2 désignent chacun, indépendamment l'un de l'autre, un groupe peu ou non électro-attracteur (peu ou non inductif-attracteur) tel que :

- un atome d'hydrogène,
- un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes

d'azote, d'oxygène, de soufre, et éventuellement substitué par un ou plusieurs groupements choisis parmi -OR, -COOR, -COR, -SH, -SR,
- OH, et les atomes d'halogène,
- un résidu polyorganosiloxane modifié ou non,
- un groupement polyoxyalkylène,

R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', - COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C1-C10,
R'3 représentant un atome d'hydrogène ou un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C1-C10.

**3.** Composition cosmétique selon la revendication 2,
**caractérisée par le fait que** le monomère cyanoacrylate est choisi parmi les monomères de formule (IV) :

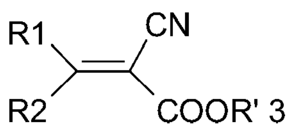

(IV)

dans laquelle R'3 représente un radical alkyle en C1-C10, alcényle en C2-C10, ou alcoxy(C1-C4) alkyle(C1-C10).

**4.** Composition cosmétique selon la revendication 3, **caractérisée par le fait que** le monomère cyanoacrylate est choisi parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle, le cyanoacrylate d'iso-amyle, le 2-cyanoacrylate d'allyle et le 2-cyanoacryale de methoxypropyle.

**5.** Composition cosmétique selon la revendication 4, **caractérisée par le fait que** le monomère cyanoacrylate est choisi parmi les cyanoacrylates d'alkyle en C6-C10.

**6.** Composition cosmétique selon la revendication 5, **caractérisée par le fait que** le monomère cyanoacrylate est choisi parmi les monomères les cyanoacrylates d'octyle de formule (V) et leurs mélanges :

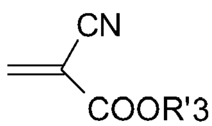

(V)

dans laquelle :

R'3 =-(CH2)7-CH3,

-CH(CH3)-(CH2)5-CH3,

-CH2-CH(C2H5)-(CH2)3-CH3,

-(CH2)5-CH(CH3)-CH3,

-(CH2)4-CH(C2H5)-CH3.

7. Composition cosmétique selon l'une quelconque des revendications précédentes, **caractérisé en ce que** le ou les monomères cyanoacrylate sont fixés de façon covalente sur des supports tels que des polymères, des oligomères ou des dendrimères.

8. Composition cosmétique selon l'une quelconque des revendications précédentes, **caractérisée en ce que** la teneur en monomère cyanoacrylate varie en une quantité allant de 0,1 à 80 % en poids, de préférence en une quantité allant de 0,2 à 60 % en poids, et encore plus préférentiellement en une quantité allant de 0,5 en 50 % en poids, par rapport au poids total de la composition.

9. Composition cosmétique selon l'une quelconque des revendications précédentes, **caractérisée par le fait que** la ou les particules organiques thermostables non colorées présentent une taille primaire moyenne en nombre comprise entre 0,1 et 30 $\mu$m, de préférence comprise entre 0,2 et 20 $\mu$m, et encore plus préférentiellement comprise entre 0,5 et 15 $\mu$m.

10. Composition cosmétique selon l'une quelconque des revendications précédentes, **caractérisée par le fait que** la ou les particules organiques est ou sont creuses ou poreuses.

11. Composition cosmétique selon la revendication 10, **caractérisée par le fait que** la ou les particules organiques sont poreuses.

12. Composition cosmétique selon la revendication 11, **caractérisée par le fait que** la ou les particules organiques poreuses présentent une surface spécifique supérieure ou égale à 1 $m^2$/g, de préférence supérieure ou égale à 2 $m^2$/g, et encore plus préférentiellement supérieure ou égale à 4 $m^2$/g.

13. Composition cosmétique selon l'une quelconque des revendications précédentes, **caractérisée par le fait que** la ou les particules organiques sont choisies parmi les poudres de polyamide, les poudres de polymères acryliques, les poudres de copolymère acryliques, les poudres de polystyrène, les poudres de polyéthylène, les microbilles de résine de silicone.

14. Composition cosmétique selon la revendication 13, **caractérisée par le fait que** la ou les particules organiques sont choisis parmi les poudres de polyamide et les poudres de polyméthylméthacrylates.

15. Composition cosmétique selon l'une quelconque des revendications précédentes, **caractérisée par le fait que** la ou les particules organiques thermostables non colorées est ou sont présentes en une quantité allant de 0,1 à 20 % en poids, de préférence en une quantité allant de 0,2 à 15 % en poids, et encore plus préférentiellement en une quantité allant de 0,5 à 10 % en poids, par rapport au poids total de la composition cosmétique.

16. Composition cosmétique selon l'une quelconque des revendications précédentes, **caractérisée par** le fait le solvant organique liquide est choisi parmi les alcools aromatiques tels que l'alcool benzylique ; les alcools gras liquides , notamment en $C_{10}$-$C_{30}$; les polyols modifiés ou non tels que le glycérol, le glycol, le propylène glycol, le dipropylène glycol, le butylène glycol, le butyle diglycol ; les silicones volatiles telles que la cylopentasiloxane, la cyclohexasiloxane, les polydiméthylsiloxanes modifiées ou non par des fonctions alkyle et/ou amine et/ou imine et/ou fluoroalkyl et/ou carboxylique et/ou betaïne et/ou ammonium quaternaire, les polydiméthylsiloxanes modifiées liquides, les huiles minérales, organiques ou végétales, les alcanes et plus particulièrement les alcanes de $C_5$ à $C_{10}$ ; les acides gras liquides, les esters gras liquides et plus particulièrement les benzoates ou les salicylates d'alcool gras et leurs mélanges.

17. Composition cosmétique selon la revendication 16, **caractérisée par le fait que** le solvant organique liquide est constitué d'un mélange de poly diméthyl siloxane alpha-omega dihydroxylé/cyclopentadiméthylsiloxane et de cyclopentadiméthyl siloxane.

**18.** Composition cosmétique selon la revendication 16 ou 17, **caractérisée par le fait que** le solvant organique représente de 0,01 à 99 %, de préférence de 50 à 99 % en poids par rapport au poids total de la composition cosmétique.

**19.** Composition cosmétique selon l'une quelconque des revendications précédentes, **caractérisée par le fait qu'**elle comprend en outre au moins un pigment.

**20.** Composition cosmétique selon la revendication 19, **caractérisée par le fait que** le ou les pigments est ou sont présents en une quantité allant de 0,1 à 50 % en poids, de préférence en une quantité allant de 0.5 à 30 % en poids, et encore plus préférentiellement en une quantité allant de 1 à 10 % en poids, par rapport au poids total de la composition.

**21.** Composition cosmétique selon l'une quelconque des revendications précédentes, **caractérisée par le fait qu'**elle comprend en outre au moins un inhibiteur de polymérisation.

**22.** Composition cosmétique selon la revendication 21, **caractérisée par le fait que le fait que** l'inhibiteur de polymérisation est un acide minéral ou organique choisi parmi l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique, l'acide trifluoroacétique, l'acide octanoïque, l'acide heptanoïque et l'acide hexanoïque.

**23.** Composition cosmétique selon la revendication 22, **caractérisée par le fait que** l'acide est l'acide acétique.

**24.** Composition cosmétique selon l'une quelconque des revendications 21 à 23, **caractérisée par le fait que** les inhibiteurs de polymérisation sont présents en une quantité allant de 10 ppm à 30 % en poids, de préférence en une quantité allant de 10 ppm à 15 % en poids, par rapport au poids total de la composition cosmétique.

**25.** Composition cosmétique selon l'une quelconque des revendications précédentes, **caractérisée par le fait que** ladite composition est anhydre.

**26.** Composition cosmétique selon l'une quelconque des revendications précédentes, **caractérisée par le fait qu'**elle comprend en outre au moins un agent nucléophile.

**27.** Composition cosmétique selon les revendications 1 à 24 et 26, **caractérisée par le fait que** l'agent nucléophile est de l'eau.

**28.** Procédé de traitement cosmétique des fibres kératiniques, **caractérisé par le fait que** l'on applique sur lesdites fibres la composition telle que définie à l'une quelconque des revendications 1 à 25 en présence d'un agent nucléophile.

**29.** Procédé de traitement selon la revendication 28, **caractérisé par le fait que** l'on applique la composition telle que définie à la revendication 26 ou 27.

**30.** Procédé de coloration capillaire selon la revendication 28, **caractérisé par le fait qu'**il comprend une première étape consistant à appliquer une composition contenant au moins un pigment sur lesdites fibres kératiniques et une seconde étape consistant à appliquer une composition cosmétique telle que définie à l'une quelconque des revendications 1 à 18 et 21 à 25, l'agent nucléophile étant présent dans la composition comprenant le pigment ou dans une composition séparée.

**31.** Utilisation d'une composition cosmétique selon l'une quelconque des revendications 1 à 27 pour le traitement des cheveux.

**32.** Dispositif à plusieurs compartiments ou kit, comprenant un premier compartiment, comprenant une composition contenant au moins un monomère cyanoacrylate polymérisable tel que défini à l'une quelconque des revendications 1 à 8 et éventuellement au moins un inhibiteur de polymérisation anionique et/ou radicalaire, et un second compartiment, comprenant une seconde composition contenant, au moins un solvant organique liquide tel que défini à l'une quelconque des revendications 16 à 18 et au moins une particule organique thermostable non colorée telle que définie à l'une quelconque des revendications 9 à 15.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2833489
- US 3527224 A
- US 3591767 A
- US 3667472 A
- US 3995641 A
- US 4035334 A
- US 4650826 A
- FR 2679771
- EP 1184426 A
- US 6225198 B
- US 5990479 A
- US 4578266 A
- US 6224622 B


### Littérature non-brevet citée dans la description


- **PR WELLS.** *Prog. Phys. Org. Chem.,* 1968, vol. 6, 111
- **JERRY MARCH.** Advanced Organic Chemistry. 151-161
- **JERRY MARCH.** Advanced Organic Chemistry. 141
- *The journal of the Americain Chemical Society,* Février 1938, vol. 60, 309
- **DABBOUSSI B.O. et al.** CdSe)ZnS core-shell quantum dots : synthesis and characterisation of a size séries of highly luminescent nanocristallites. *Journal of phisical chemistry B,* 1997, vol. 101, 9463-9475
- **PENG, XIAOGANG et al.** Epitaxial Growth of highly Luminescent CdSe/CdS core/shell nanocrystals with photostability and electronic accessibility. *Journal of the American Chemical Society,* vol. 119 (30), 7019-7029